# EUROPEAN PATENT APPLICATION

(11) **EP 4 451 278 A1**
(43) Date of publication of application: **23.10.2024**
(21) Application number: 21967749.9
(22) Date of filing: 17.12.2021
(51) Int. Cl.: G16B 50/30

(54) **METHOD AND SYSTEM FOR STORING INFORMATION USING DNA**

(71) Applicant: BGI Shenzhen, Shenzhen, Guangdong 518083 (CN)
(72) Inventor: SHEN, Yue, Shenzhen, Guangdong 518083 (CN); PING, Zhi, Shenzhen, Guangdong 518083 (CN); ZHANG, Haoling, Shenzhen, Guangdong 518083 (CN); ZHANG, Wenwei, Shenzhen, Guangdong 518083 (CN)
(74) Representative: Rentsch Partner AG
(86) International application number: PCT/CN2021/139186
(87) International publication number: WO 2023/108616

(57) **Abstract**

Provided are a method and system for storing information using DNA. The method comprises: 1) obtaining a DNA fragment attribute library, wherein the DNA fragment attribute library comprises a plurality of DNA molecules, and comprises a retrieval table of storage object attributes and the DNA molecules, and each DNA molecule comprises double-stranded DNA, which represents the storage object attribute, and a single-stranded sticky end for connection; 2) on the basis of the retrieval table, encoding information to be stored, so that same is directly mapped to a corresponding DNA molecule in the DNA fragment attribute library; 3) acquiring corresponding DNA fragments from the DNA fragment attribute library, and connecting same, so as to obtain a connected DNA molecule; and 4) storing the connected DNA molecule. The method and system can be extended to different data types and different application scenarios, and prevent an oligonucleotide sequence from being repeatedly synthesized during storage, thereby greatly reducing storage cost.

## Description

### TECHNICAL FIELD

The present disclosure relates to the field of biotechnology and bioinformatics, and more specifically, provides a method and system for storing information using DNA.

### BACKGROUND

With the development of modern science and technology, especially the Internet, global data are growing exponentially. The ever-increasing data volume puts a higher and higher demand on storage technology. However, traditional storage technologies, such as magnetic tapes and optical disks, are increasingly unable to meet the current data storage needs due to their low storage density and limited storage time. The recent development of DNA data storage technology provides a new approach to solve the problem. Compared with traditional storage media, DNA, as a storage medium for information storage, is characterized by long storage time (up to several thousand years or more, more than 100 times that of an existing magnetic tape and an optical disk), high storage density (up to ~10⁹ Gb/mm³, more than 10 million times that of a magnetic tape and an optical disk) and good storage security.

In 2012, Church *et al.* proposed to use DNA for the storage of binary digital information. The adopted method is to extract the binary information of files and divide it into small fragments, convert it into DNA format using specific conversion codes, and artificially synthesize it de novo in the form of oligonucleotides (a length of ~200 bases), so as to complete the information storage. When the information is read, the DNA information is interpreted by sequencing and decoded into the original files by reverse operation using the conversion codes.

In 2017, Church *et al.* demonstrated that the CRISPR system is capable of encoding information as complex as a digital video of human. They introduced DNA into *E. coli* at a rate of one frame per day over five days, and subsequently sequenced the CRISPR regions in the bacterial population, so as to restore the images. Since the CRISPR system adds DNA fragments sequentially, the location of each fragment in the array can be used to determine the original frame to which a fragment belongs. From the perspective of industrialization, the efficiency of the CRISPR-based system is very low, even lower than that of oligonucleotide synthesis.

In 2019, Takahashi *et al.* firstly publicly presented a fully automated microfluidic-based DNA data storage system using microfluidics. The system firstly requires DNA to be synthesized on demand. To extract the data from DNA, the system will firstly add some chemicals to the DNA for pretreatment, introduce the liquid into the rest of the system by using a microfluidic pump, read the DNA sequences, and convert it back into a computer-understandable language. The system has to use liquid to transfer molecules, and is therefore prone to contamination of the fluid pathways, which would reduce the correct yield. Also, the pre-operation of DNA synthesis is necessary for the system, which would result in low efficiency and non-reusability.

Generally, DNA data storage comprises the following steps: 1) encoding: converting the binary codes 0/1 of computer information into A/T/C/G of DNA sequence information; 2) synthesis: synthesizing the corresponding DNA sequences by using DNA synthesis technology, and preserving the obtained chemical DNA molecules in an *in vitro* medium or living cells; 3) sequencing: reading the DNA sequences of the stored DNA molecules by using the sequencing technology; 4) decoding: converting the DNA sequences obtained by sequencing into binary codes 0/1 and further into computer information by using a process corresponding to the encoding process in 1). The main disadvantages of such DNA data storage are as follows: (1) not reusable: for a traditional information file, DNA for storage needs to be synthesized separately and the synthesized DNA molecule cannot be used for the storage of other information files; (2) difficult to realize data processing: it is difficult to modify, delete and expand the stored original information; (3) difficult to being proceduralized: for a special storage need, it needs to be customized de novo; (4) unstable performance: even a slight change in the length of a data region or the file format would easily lead to an encoding algorithm requiring more logical redundancy than its theoretical situation; (5) not easy to categorize: the existing classification and archiving methods can only be achieved by designing different primer-binding regions. Therefore, limited by the DNA synthesis techniques (oligonucleotide synthesis) commonly used in DNA storage, the existing DNA storage requires much higher cost and more time than the traditional storage technologies. In addition, it is difficult to reuse the conventional DNA synthesis strategies reasonably. When re-encoding binary information, it is necessary to synthesize DNA de novo, which greatly increases the time and economic cost of the existing DNA storage.

Therefore, for efficient DNA data storage, there is a need in the art to develop a DNA data storage technology in which synthetic sequences can be reused.

### SUMMARY

The present disclosure aims to provide a DNA data storage scheme, wherein DNA data storage is realized by pre-synthesizing an attribute library of DNA fragments required for unstructured data and using only a small portion of each fragment in single information storage.

Therefore, in a first aspect, the present disclosure provides a method for storing information using DNA, comprising:
1) obtaining an attribute library of DNA fragments, wherein the attribute library of DNA fragments comprises a plurality of DNA molecules and a retrieval table of the attributes of stored objects and the DNA molecules, and each DNA molecule comprises double-stranded DNA representing the attribute of a stored object and a single-stranded sticky end for ligation;
2) encoding the information to be stored into the corresponding DNA molecules directly mapping to the attribute library of DNA fragments based on the retrieval table;
3) obtaining the corresponding DNA fragments from the attribute library of DNA fragments and ligating the corresponding DNA fragments to obtain the ligated DNA molecules;
4) storing the ligated DNA molecules.

In one embodiment, the method further comprises: 5) adding, deleting, modifying or searching the stored DNA molecules.

In one embodiment, adding, deleting, modifying or searching the stored DNA molecules are performed by CRISPR technology or DNA strand displacement.

In one embodiment, the method further comprises: 6) obtaining the sequencing information of the stored DNA molecules and restoring the stored information based on the sequencing information and the retrieval table.

In one embodiment, in 1), there is a plurality of the attribute libraries of DNA fragments that correspond to different attributes of the stored objects.

In one embodiment, in 1), each DNA molecule in the retrieval table corresponds to one or more locations in the attribute library of DNA fragments.

In one embodiment, in 1), the double-stranded DNA representing the attributes of the stored objects is 8-32 bp in length.

In one embodiment, in 1), the single-stranded sticky end comprises 4-8 nucleotides.

In one embodiment, in 2), the information to be stored is unstructured data.

In one embodiment, in 4), the ligated DNA molecules are placed in a container or introduced into an organism.

In a second aspect, the present disclosure provides a system for storing information using DNA, comprising:
an attribute library of DNA fragments, wherein the attribute library of DNA fragments comprises a plurality of DNA molecules and a retrieval table of the attributes of stored objects and the DNA molecules, and each DNA molecule comprises double-stranded DNA representing the attribute of a stored object and a single-stranded sticky end for ligation;
a retrieval unit, which is configured to encode the information to be stored into the corresponding DNA molecules directly mapping to the attribute library of DNA fragments based on the retrieval table;
a ligation unit, which is configured to ligate the corresponding DNA fragments obtained from the attribute library of DNA fragments to obtain the ligated DNA molecules;
a storage unit, which is configured to store the ligated DNA molecules.

In one embodiment, the system further comprises an editing unit, which is configured for adding, deleting, modifying or searching the stored DNA molecules.

In one embodiment, adding, deleting, modifying or searching the stored DNA molecules are performed by CRISPR technology or DNA strand displacement.

In one embodiment, the system further comprises a sequencing unit and a decoding unit, wherein the sequencing unit is configured to obtain the sequencing information of the stored DNA molecules and the decoding unit is configured to restore the stored information based on the sequencing information and the retrieval table.

In one embodiment, there is a plurality of the attribute libraries of DNA fragments that correspond to different attributes of the stored objects.

In one embodiment, each DNA molecule in the retrieval table corresponds to one or more locations in the attribute library of DNA fragments.

In one embodiment, the double-stranded DNA representing the attributes of the stored objects is 8-32 bp in length.

In one embodiment, the single-stranded sticky end comprises 4-8 nucleotides.

In one embodiment, the information to be stored is unstructured data.

In one embodiment, in the storage unit, the ligated DNA molecules are placed in a container or introduced into an organism.

The present disclosure proposes to pre-synthesize an attribute library of DNA fragments (corresponding to a printer cartridge) required for unstructured data, and only using a small portion of each fragment (corresponding to an ink droplet) for every information storage. The conversion process from information to DNA is realized only by ligating the selected DNA fragments with different attributes via sticky ends, which is thus easy for expansion and procedural operation. The DNA storage method of the present disclosure can be applied to different data types and different scenarios, enabling the storage process while avoiding repeated synthesis of oligonucleotide sequences, which would greatly reduce storage cost.

### BRIEF DESCRIPTION OF THE DRAWINGS

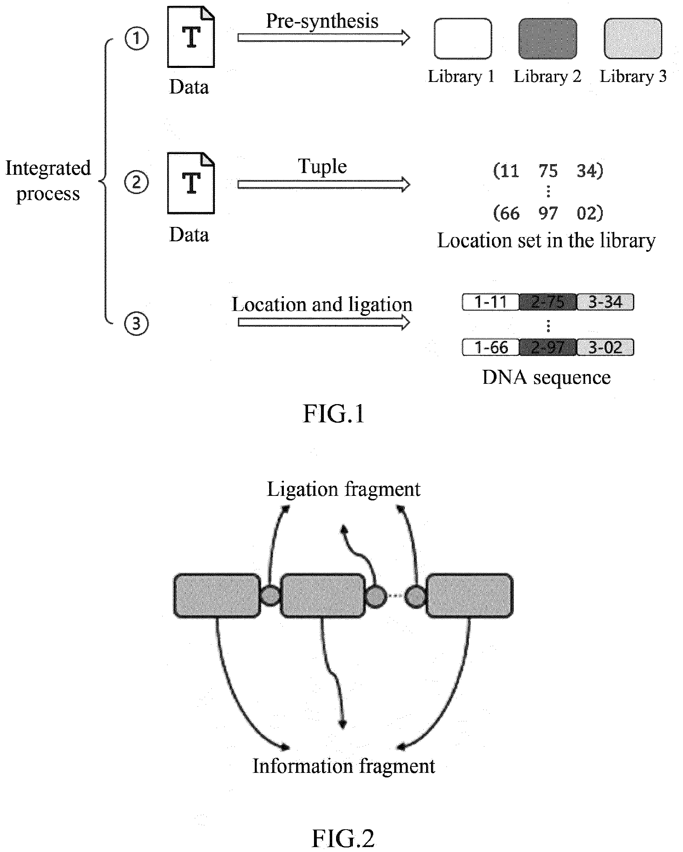

Figure 1 shows a pre-synthesized library based DNA storage process according to an embodiment of the present disclosure. Pre-synthesizing an attribute library as required; finding the attribute of the information of each unit based on the information of the data to be stored, and determining its molecule in the library based on the attribute requirements; locating the molecules, extracting the molecules, and ligating the molecules successively or simultaneously to create DNA sequences.
Figure 2 shows a schematic diagram of the structure of a DNA sequence comprising information fragments and ligation fragments according to the present disclosure. The ligated DNA sequence is composed of a plurality of information fragments and ligated fragments in turn.

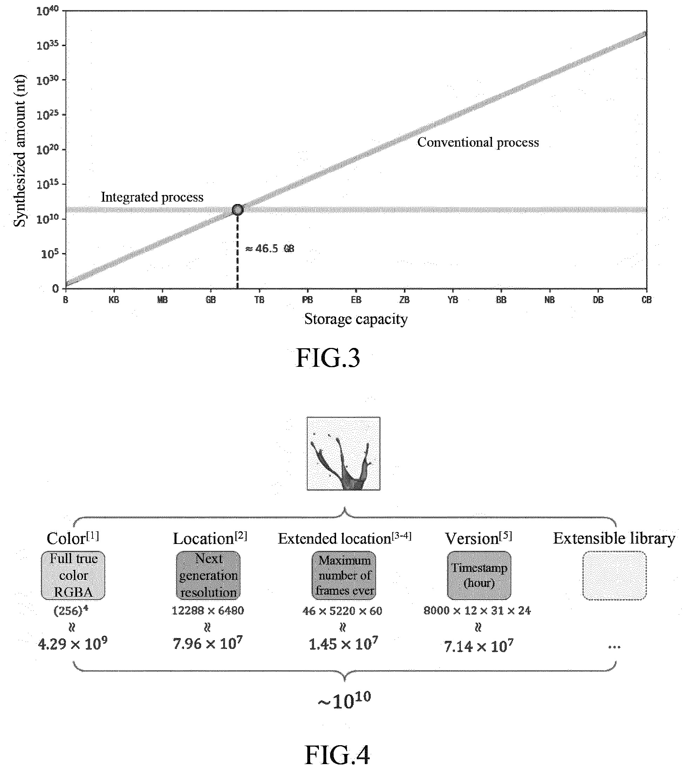

Figure 3 shows a comparison between the process according to the present disclosure and a conventional process. In the conventional process, the highest ratio of storage capacity to synthesized amount is 2:1, that is, 1 nucleotide would be required to synthesize 2 bits of information, and thus the synthesized amount is at least half of the storage capacity. In one example, the process of the present disclosure requires a pre-synthetic amount of 20*10^10 nucleotides. Based on function calculations, at 46.5 GB, the synthesized amount of the integrated process is equal to that of the conventional process; after exceeding 46.5 GB, the synthetic amount of the integrated process is much less than that of the conventional process.
Figure 4 illustrates the picture-based library capacity estimation. The picture data is temporarily divided into 4 libraries, wherein the color library is set according to the RGBA format with the richest colors. There are 256 kinds of red, green, blue and transparency, respectively, so a total of 256^4 (about 4.29* 10^9) types of molecules. The location library has a size of 12288*6480 (about 7.96*10^7) types of molecules calculated on the basis of the next generation video resolution, i.e., 6K resolution. The extended location library is in reference to the requirement and representation of video formats (frame), to the longest film in the world (5220 minutes) and the minimum number of frames indiscernible by eyes (46 frames per second), and thus it is inferred that the extended location library requires about 8.64*10^8 types of molecules. It is inferred that the version library requires a total of 7.14*10^7 types of molecules by using hour as the minimum unit, supported by about 8,000 years before 10,000 AD, about 365 days per year and 24 hours per day. In summary, the total library capacity requires a total of about 10^10 types of molecules to be synthesized.

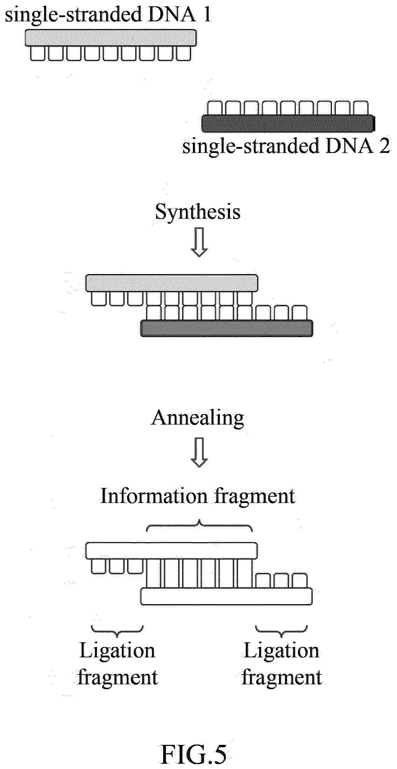

Figure 5 illustrates the process of creating a single DNA molecule. For a single molecule, two single strands of DNA are firstly synthesized and each single strand comprises information fragments and ligation fragments; secondly, by using annealing technology, the information fragments of two DNA sequences are ligated based on the principle of complementary base pairing to create a single DNA molecule.

### DETAILED DESCRIPTION

To achieve rapid instantiation, standardization, and reusability of DNA storage, the inventors propose an information storage process using DNA based on a pre-synthesized library, which is referred to as an integrated process, as shown in Figure 1. The integrated process system of the present disclosure mainly consists of three steps, which are specifically described as follows. (1) A pre-synthesized DNA library comprises two or more attribute libraries, each of which represents information of the stored objects, including attributes such as color, location, and time information. In the present disclosure, the information can be represented by the attributes, and a plurality of attributes can be stringed together to represent information integrally. The information can be one or more of text, picture, audio and video. For example, for textual information, the attributes may include words, expressions, phrases, punctuations, etc. The size of an attribute library can vary for different information, as long as it can represent the information in whole or in part. In one example, different information can share an attribute library, such as information in the form of pictures and videos. Each location in the attribute library of DNA fragments comprises one type of DNA molecules in the attribute library. Two locations may comprise the same type of DNA molecule if a redundancy is desired. Each molecule comprises a double-stranded DNA representing the attributes of a stored object (hereinafter referred to as an information fragment) and a single-stranded sticky end (hereinafter referred to as a ligation fragment) for ligation, as shown in Figure 2. (2) The information is directly mapped to the corresponding locations in the attribute library, and two or more consecutive DNA molecules representing the information are ligated by using DNA ligation means including but not limited to ligase. (3) The ligated molecules are placed in a test tube or introduced in an organism.

There is no need to repeatedly synthesize oligonucleotide sequences in the process of the example. Calculated from the perspective of the synthesized amount of nucleotides, as shown in Figure 3, when more than 46.5 GB information needs to be synthesized, the synthesized amount of the integrated process of the present disclosure would be less than that of a conventional process. With the further increase of social demand for cold storage, the bit of the information to be synthesized can be up to the level of EB or even higher, which would be far more than 46.5 GB.

At the same time, compared with the synthesis of long strands of oligonucleotide sequences, ligating different information fragments by using ligation fragments would improve the overall efficiency and reduce the error rate to a certain extent. Through CRISPR, strand displacement reaction, and other special treatments, the ligation fragments can realize a customized function such as modification in a specific part or synchronized load.

In the present disclosure, the creation of a library is illustrated as follows: the specific attributes of unstructured data can be divided into two or more attribute libraries, and the required library capacity of each attribute library can be calculated based on the specific reference attribute settings. As shown in Figure 4, taking a picture as an example, its attributes can be approximately divided into a color library, a location library, an extended location library, a version library, etc. The total library capacity of these attribute libraries is 10^10. The synthesis of 10 billion types of molecules can be effectively accomplished based on high-throughput DNA synthesis technology. TWIST Bioscience is known to have a throughput of 1 million and can synthesize 10 billion types of molecules.

In the present disclosure, in order to reduce the systematic error of a DNA library, the information fragments are required to meet certain biochemical limitations, so as to be compatible with the stability of upstream and downstream biochemical techniques. The length of DNA sequences that satisfy both the biochemical limitations and the total library capacity can be found through large-scale screening computer, and the limitations include but are not limited to GC content, single base repeat length, specific sequences, Hamming distance, editing distance, and the like. Further, the anti-interference ability of DNA sequences can be improved by, for example, limiting the minimum difference between the DNA sequences in each attribute library to 3 nucleotides (Hamming distance greater than or equal to 3). By a customized clustering algorithm, two or more attribute libraries that meet the above requirements can be obtained.

In the present disclosure, the ligation fragments (sticky ends) also need to be reasonably designed to ensure yield and efficiency of the integrated process. Firstly, DNA sequences of the ligation fragments must not be present in the information fragments, in case that the ligation fragments are misidentified in possible subsequent biochemical reactions. Secondly, since a ligation fragment is single-stranded, it is necessary to prevent the single strand itself from forming a special secondary structure, thus affecting the efficiency of the ligation. Finally, the ligation fragments of different attributes should be very different and the ligation fragments of unrelated attributes should not be ligated. Through computerized screening and calculating, a ligation library that meets the requirements could be obtained.

In the present disclosure, the specific structure and creation process of a single DNA molecule is shown in Figure 5. The middle part of each molecule is a double-stranded information fragment, with a single-stranded fragment ligated to the front and rear thereof, respectively. The double-stranded information fragment can have a length set as desired, preferably 8-32 bp. The length of the single-stranded ligation fragment can be 4-8 nucleotides. For example, the length of the information fragment (double strand) is 20 nucleotides, and that of the ligation fragment (single strand) is 6 nucleotides. A single strand with 26 nucleotides is firstly synthesized, then the required reverse complementary strand is synthesized, and finally they are complementarily paired by using annealing technology to complete the synthesis of the molecule. Each molecule is in the pattern of "ligation fragment + information fragment + ligation fragment".

In the present disclosure, the use of a library is illustrated as follows: The combination of two attributes of unstructured data for DNA storage is also accomplished by using annealing technology to allow complementary pairing of the ligation fragments, and thus to complete the ligation between the attributes, which is similar to the process of synthesizing molecules. The combination of information fragments and ligation fragments constitutes a single attribute combination of unstructured data, as shown in Figure 2. In the case of picture information storage, it can represent the color information of a certain location.

In the present disclosure, the ligation between the attributes can be performed successively, simultaneously or in an arbitrary order. For example, multiple (more than two) attributes can be ligated in parallel based on the specificity of the ligation fragments. Parallel processing can further accelerate the DNA storage.

In the present disclosure, DNA molecules representing the attributes of the stored objects can be processed, for example, including but not limited to: 1) excising specific DNA sequences using a CRISPR system for deleting information; 2) using DNA strand displacement reaction for the capture of the sequences within a specific Hamming distance via hybridization to realize information process, such as non-destructive random read and categorization; 3) using sequence-specific magnetic beads for capturing DNA sequences to realize random read of information; 4) using capture probes and other means to search specific information based on the sequence similarity.

Therefore, in terms of yield, the integrated process of the present disclosure possesses higher robustness (i.e. lower error rate); in terms of efficiency, the integrated process of the present disclosure completes the conversion from traditional information to DNA by ligation, while the conventional process requires ligating nucleotides successively to complete the conversion, which is a huge time-cost improvement; in terms of energy consumption, the energy required for ligating DNA is much lower than that required for synthesizing DNA, therefore, the integrated process of the present disclosure meets the requirements of energy consumption goals, such as carbon peak and carbon neutrality.

In the present disclosure, the unstructured data refer to data that has an irregular or incomplete structure, does not have predefined data model, and is not suitable to be represented by two-dimensional logical tables of database, including office documents, texts, pictures, XML, HTML, various reports, images, audio/video information, etc. in all formats.

In the present disclosure, the attributes refer specifically to attributes of unstructured data. In the case of a picture, for example, the attributes include the location and color of each pixel and the version information of the picture. In the case of a video, the attributes further include the number of frames of the video in which the picture is located, parameter information of the audio frame corresponding to the picture frames, etc. In the present disclosure, a frame is a single image, the smallest unit in an image animation, equivalent to each shot in a filmstrip. A frame is a static image, and consecutive frames form an animation, such as television images.

In the present disclosure, biochemical limitations generally include the maximum number of single base repeats and GC content. If the DNA molecules are stored *in vivo,* it is necessary to remove cleavage sites of restriction endonuclease, prevent the presence of Coding Sequences (CDS) and the like. For long read sequencing, certain specific sequences that would affect electrical signals may also be removed.

In the present disclosure, a data set can be divided into different classes or clusters according to a specific criterion (such as a distance criterion) by a clustering algorithm, so that the data objects in a same cluster are as similar as possible, and at the same time the data objects not in a same cluster are as different as possible. That is, after clustering, data of a same class should be gathered as much as possible, and data of different class should be separated as much as possible. For example, Hamming distance can represent the number of positions at which the corresponding nucleotides are different between two DNA fragments.

In the present disclosure, a cut in two single-stranded DNAs is called a sticky end, which has several protruding nucleotides complementarily paired with each other. In the present disclosure, a reverse complementary strand refers to a reverse, a complement or a reverse complement counterpart of a DNA sequence. In the present disclosure, molecules in a pre-synthesized library are ligated by means including but not limited to ligases, so as to obtain a target DNA sequence. In the present disclosure, a ligation fragment may include a common restriction endonuclease site sequence or other preset sequences.

In the present disclosure, CRISPR is a gene editing system. Viruses can integrate their own genes into a bacterium and use cellular tools of the bacterium to serve for their gene replication. Bacteria have evolved a CRISPR-Cas9 system in order to eliminate foreign invasive genes of viruses. Bacteria can quietly excise viral genes from their own genome by using the system, which is a unique immune system of bacteria and an acquired immune system of archaea and bacteria to resist the invasion of foreign genetic materials such as viruses. In the present disclosure, potentially advanced operations (accomplished by using CRISPR technology or DNA strand displacement) that may be involved herein include, but are not limited to: 1) modification of information fragments by using CRISPR technology; 2) non-destructive access to information fragments by using DNA strand displacement technology; 3) access, modification or deletion of information fragments by using sequence-specific magnetic beads. For example, adding, deleting, modifying (updating) or searching another attribute is performed based on a particular attribute. In the present disclosure, DNA strand displacement, also known as Toehold-mediated strand displacement, is an enzyme-free molecular tool for exchanging one DNA or RNA strand (output) with another DNA or RNA strand (input). It is based on the hybridization of two complementary DNA or RNA strands via Watson-Crick base pairing (A-T/U and C-G) and utilizes a process known as branch migration.

### Example

In a specific example, the particular steps of creating a library are described by using a picture as an example. It should be understood that the present disclosure can be used for any unstructured data, and it is only an example for a picture here. The storage object in the example is limited to be picture data of unstructured data. The picture data are temporarily divided into 4 libraries, as shown in Figure 4, wherein the color library is set according to the RGBA format with the richest colors. There are 256 kinds of red, green, blue and transparency, respectively, so a total of 256^4 (about 4.29*10^9) types of molecules. The location library has a size of 12288*6480 (about 7.96*10^7) types of molecules calculated on the basis of the next generation video resolution, i.e., 6K resolution. The extended location library is in reference to the requirement and representation of video formats (frame), to the longest film in the world (5220 minutes) and the minimum number of frames indiscernible by eyes (46 frames per second), and thus it is inferred that the extended location library requires about 1.44*10^7 types of molecules. It is inferred that the version library requires a total of 7.14*10^7 types of molecules by using hour as the minimum unit, supported by about 8,000 years before 10,000 AD, about 365 days per year and 24 hours per day. In summary, the total library capacity requires a total of about 10^10 types of molecules to be synthesized.

Based on the common biochemical limitations in DNA storage, the information fragments are set as follows: (1) the GC content is between 40% - 60%; (2) the length of consecutive single base repeat (such as AA...A) is no more than 3; (3) no palindromic and complementary repeats is more than 4 in length; (4) Hamming distance of sequences in each attribute library is required to be greater than or equal to 4, so that the single base error can be corrected. Approximately 6.1*10^11 combinations of information fragments with a length of 20 nucleotides that meet the biochemical limitations are obtained by computer calculation, far exceeding the established requirement of 10^10 molecular combinations.

Based on the experience of actual experiment procedures, the requirements for the ligation regions are as follows: (1) the length is 4 to 8 nucleotides; (2) no special secondary structure is formed through experimental verification; (3) the formation of the ligation is not influenced through experimental verification; (4) distinguishable difference presents, and Hamming distance is greater than or equal to 4; (4) no overlap is with the information fragments. The ligation fragments with a length of 6 nucleotides and with a library capacity of 6 to 8 is eventually obtained by computer calculation. Further requirements (or limitations) can be revised on the basis of these conditions.

Based on the above requirements for the information regions and ligation regions, the synthesis of the library was performed. Firstly, a single strand of 26 nucleotides (comprising an information region of 20 nucleotides and a ligation region of 6 nucleotides) is synthesized, then the required reverse complementary strand thereof is synthesized, and finally, the molecule is synthesized by complementarily pairing the above two strands using annealing technology. Each molecule conforms to the pattern of "ligation fragment + information fragment + ligation fragment".

Synthesis (addition) process: The basic attributes of a pixel are synthesized, taking color information + pixel location as an example, as follows. It was assumed that RGBA color [0,0,0,255] (corresponding to a DNA sequence of AAATTTGGGTGGGTGGTGTG, a ligation region of ACGTAC) on the pixel location [100,100] (corresponding to a DNA sequence of AATTTGGGCTTGCCGCTCGC, a ligation region of GTACGT) was recorded. Firstly, the corresponding location based on the information was obtained, and the sequence-containing solution (comprising the corresponding DNA sequences) at the location was obtained. Through annealing process, the ligation regions of two single strands were ligated to obtain the double-stranded DNA with a length of 46 nucleotides (AAATTTGGGTGGGTGGTGTG-ACGTAC-AATTTGGGCTTGCCGCTCGC).

Search process: A conventional process is to capture the corresponding DNA sequences by using sequence-specific magnetic beads. For example, the inventor intended to search for all areas with a color of [0,0,0,255] in a certain picture. Reverse complementary sequence (single strand) of the sequence of the color [0,0,0,255] information region was designed. The double-stranded DNA was dissociated into single strands by denaturation process of PCR, followed by binding the DNA sequences of 46 nucleotides in length to the magnetic beads by annealing. The search required for the case was completed by sequencing the sequence on the magnetic beads and collecting the specific information of the last 20 nucleotides. Further, the above search could be completed by DNA strand displacement reaction. Firstly, the corresponding single strand (displacement strand) was designed, and then the original strand was stripped by denaturation. The collected original strand was sequenced to complete the search required for the case.

Modification process: The simplest modification strategy is to further add version information to each sequence. In the actual sequencing process, it is only needed to record the corresponding information of the latest version. Taking version information [1] as an example, the corresponding DNA sequence was GCAAGTCATTGAGATCCTAC and the ligation region was TGTGAC. After the synthesis of the corresponding sequence, the double-stranded DNA sequence with a length of 72 nucleotides was obtained: AAATTTGGGTGGGTGGTGTG-ACGTAC-AATTTGGGCTTGCCGCTCGC-TGTGAC-GCA AGTCATTGAGATCCTAC. During the sequencing process, if a longer DNA sequence was found, the program would record it firstly to obtain the required DNA sequence, and then determine whether to replace the corresponding content according to the version information. CRISPR-based modification simply involves cutting the corresponding information and adding in a new synthesized DNA molecule carrying the modified information. For modifying the color information at a certain location, a corresponding guide RNA (gRNA) was designed and DNA molecules carrying that information were cut by using the cutting properties of CRISPR. The cut molecules could not be amplified in the subsequent data recovery and could be distinguished from the original information (due to the significant change in length) by means including, but not limited to, gel electrophoresis and high-performance liquid chromatography (HPLC).

Deletion process: It is consistent with the simple strategy in the modification process. Because of the addition of version information, DNA sequences with earlier version information or without version information could be determined as deleted information at the computer level and thus would not be further processed. In addition, the deletion could also be carried out by a magnetic bead-based method. Specifically, sequence-specific magnetic beads were designed to adsorb DNA sequences of the information to be deleted, and then the selected DNA sequences were deleted by filtration and purification.

## Claims

1. A method for storing information using DNA, comprising:
1) obtaining an attribute library of DNA fragments, wherein the attribute library of DNA fragments comprises a plurality of DNA molecules and a retrieval table of the attributes of stored objects and the DNA molecules, and each DNA molecule comprises double-stranded DNA representing the attribute of a stored object and a single-stranded sticky end for ligation;
2) encoding the information to be stored into the corresponding DNA molecules directly mapping to the attribute library of DNA fragments based on the retrieval table;
3) obtaining the corresponding DNA fragments from the attribute library of DNA fragments and ligating the corresponding DNA fragments to obtain the ligated DNA molecules;
4) storing the ligated DNA molecules.

2. The method of claim 1, further comprising: 5) adding, deleting, modifying or searching the stored DNA molecules.

3. The method of claim 2, wherein adding, deleting, modifying or searching the stored DNA molecules are performed by CRISPR technology or DNA strand displacement.

4. The method of any one of claims 1-3, further comprising: 6) obtaining the sequencing information of the stored DNA molecules and restoring the stored information based on the sequencing information and the retrieval table.

5. The method of any one of claims 1-3, wherein there is a plurality of the attribute libraries of DNA fragments that correspond to different attributes of the stored objects.

6. The method of any one of claims 1-3, in 1), each DNA molecule in the retrieval table corresponds to one or more locations in the attribute library of DNA fragments.

7. The method of any one of claims 1-3, in 1), the double-stranded DNA representing the attributes of the stored objects is 8-32 bp in length.

8. The method of any one of claims 1-3, in 1), the single-stranded sticky end comprises 4-8 nucleotides.

9. The method of any one of claims 1-3, in 2), the information to be stored is unstructured data.

10. The method of any one of claims 1-3, in 4), the ligated DNA molecules are placed in a container or introduced into an organism.

11. A system for storing information using DNA, comprising:
an attribute library of DNA fragments, wherein the attribute library of DNA fragments comprises a plurality of DNA molecules and a retrieval table of the attributes of stored objects and the DNA molecules, and each DNA molecule comprises double-stranded DNA representing the attribute of a stored object and a single-stranded sticky end for ligation;
a retrieval unit, which is configured to encode the information to be stored into the corresponding DNA molecules directly mapping to the attribute library of DNA fragments based on the retrieval table;
a ligation unit, which is configured to ligate the corresponding DNA fragments obtained from the attribute library of DNA fragments to obtain the ligated DNA molecules;
a storage unit, which is configured to store the ligated DNA molecules.

12. The system of claim 11, wherein the system further comprises an editing unit, which is configured for adding, deleting, modifying or searching the stored DNA molecules.

13. The system of claim 12, wherein adding, deleting, modifying or searching the stored DNA molecules are performed by CRISPR technology or DNA strand displacement.

14. The system of any one of claims 11-13, wherein the system further comprises a sequencing unit and a decoding unit, wherein the sequencing unit is configured to obtain the sequencing information of the stored DNA molecules and the decoding unit is configured to restore the stored information based on the sequencing information and the retrieval table.

15. The system of any one of claims 11-13, wherein there is a plurality of the libraries of DNA fragments that correspond to different attributes of the stored objects.

16. The system of any one of claims 11-13, wherein each DNA molecule in the retrieval table corresponds to one or more locations in the attribute library of DNA fragments.

17. The system of any one of claims 11-13, wherein the double-stranded DNA representing the attributes of the stored objects is 8-32 bp in length.

18. The system of any one of claims 11-13, wherein the single-stranded sticky end comprises 4-8 nucleotides.

19. The system of any one of claims 11-13, wherein the information to be stored is unstructured data.

20. The system of any one of claims 11-13, wherein in the storage unit, the ligated DNA molecules are placed in a container or introduced into an organism.
